## Europäisches Patentamt

## European Patent Office

## Office européen des brevets

(19)

(11) Veröffentlichungsnummer: **0 179 976**
**B1**

(12)

# EUROPÄISCHE PATENTSCHRIFT

(45) Veröffentlichungstag der Patentschrift:
04.10.89

(51) Int. Cl.⁴: **B 67 C 3/26,** B 67 C 3/34

(21) Anmeldenummer: **85105210.0**

(22) Anmeldetag: **29.04.85**

(54) **Kontinuierlich arbeitende Befüllungsvorrichtung.**

(30) Priorität: **02.10.84 IT 358984**

(43) Veröffentlichungstag der Anmeldung:
**07.05.86 Patentblatt 86/19**

(45) Bekanntmachung des Hinweises auf die Patenterteilung:
**04.10.89 Patentblatt 89/40**

(84) Benannte Vertragsstaaten:
**AT BE CH DE FR GB IT LI LU NL SE**

(56) Entgegenhaltungen:
**BE-A-834 961**
**DE-A-2 166 119**
**DE-A-3 213 996**
**GB-A-2 154 992**
**US-A-3 205 920**
**US-A-3 559 702**

(73) Patentinhaber: **SIMONAZZI A. & L. S.p.A., Via la Spezia 241/A, I-43016 Parma (IT)**

(72) Erfinder: **Simonazzi, Adriano, Dr., Via la Spezia n. 241a, I-43016 Parma (IT)**

(74) Vertreter: **Beszédes, Stephan G., Dr. Patentanwalt, Münchener Strasse 80a Postfach 1168, D-8060 Dachau (DE)**

LIBER, STOCKHOLM 1989

## Beschreibung

Die Erfindung betrifft eine kontinuierlich arbeitende Befüllungsvorrichtung mit einem rotierenden Tisch und mit einer Mehrzahl von Füllköpfen die über Pneumatikglieder mit dem Rand des zu füllenden Behälters dichtend in Anlage bringbar sind.

Eine derartige Befüllungsvorrichtung ist aus der US-A-3 559 702 bekannt. Die vorbekannte Vorrichtung besitzt eine Mehrzahl von Füllköpfen, die über Pneumatikglieder mit dem Rand des zu füllenden Behälters dichtend in Anlage bringbar sind.

Aus der DE-OS-1 956 463 ist eine Maschine zum Füllen von Flaschen bekannt, bei der ein Kolben vorhanden ist, der in vertikaler Richtung beweglich ist, der vor dem Füllvorgang nach unten bewegt wird und nach dem Füllvorgang wieder nach oben bewegt wird.

Mit den vorbekannten Rotationsbefüllungsvorrichtungen können hohe Produktionsgeschwindigkeiten nicht erreicht werden, wenn damit dünnwandige, rohrförmige Behälter mit einem sehr empfindlichen Rand der Einfüllöffnung gefüllt werden sollen. Die meisten Nachteile beruhen dabei auf der Tatsache, daß an dem schwachen Rand der Einfüllöffnung zwecks Sicherstellung der für das Ablaufen der Befüllungsprozesse erforderlichen Dichtigkeit eine nicht adäquate axiale Kraft ausgeübt wird, die den äußerst empfindlichen oberen Rand ebenso beschädigt wie die schwache zylindrische Struktur, die eine sehr geringe Wandstärke aufweist.

Aufgabe der Erfindung ist es daher, die axiale Kraft zum Abdichten des Randes der Einfüllöffnung zu optimieren und zu steuern, um weder den Rand noch die zylindrische Struktur von sehr dünnwandigen Behältern während des Füllens zu beschädigen.

Erfindungsgemäß wird diese Aufgabe bei einer kontinuierlich arbeitenden Befüllungsvorrichtung der eingangs angegebenen Art dadurch gelöst, daß die Pneumatikglieder mit Druckluftbehältern verbunden sind, deren Innendruckverhältnis gesteuert und vorherbestimmbar ist, indem nur auf ein einziges zentrales, dieses steuerndes Bauteil eingewirkt wird. Hierdurch ist es möglich, die Dichtigkeit an dem Rand der Öffnung eines zu füllenden Behälters abhängig vom Ablauf des Füllvorganges zu optimieren. Weiterhin ist dadurch die Steuerung der Optimierung der Dichtigkeit zentral zusammengefaßt und während der kontinuierlichen Rotation der Befüllungsvorrichtung mit hoher Geschwindigkeit ausführbar.

Vorteilhafte Weiterbildungen sind in den Unteransprüchen beschrieben.

Ausführungsbeispiele der Erfindung werden nachstehend an Hand der Zeichnungen näher erläutert. Es zeigen:

Fig. 1　einen Axialschnitt eines Pneumatikgliedes, das einem einzelnen Befüllungshahn zugeordnet ist;

Fig. 2　eine Schnittansicht eines koaxial zum entsprechenden Hahn eingebauten Pneumatikgliedes;

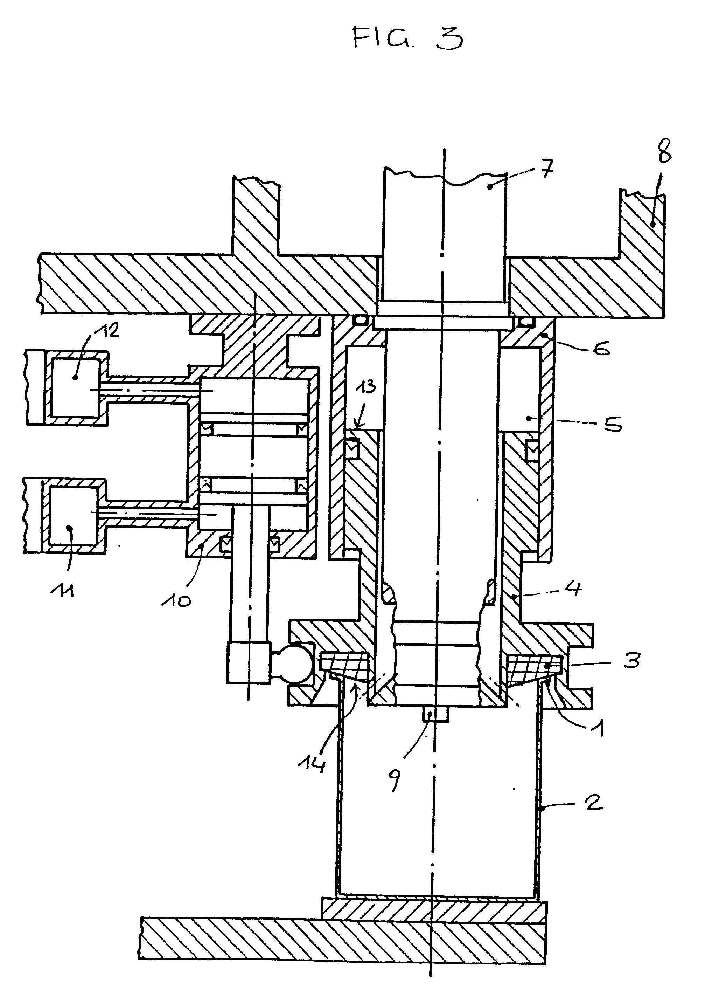

Fig. 3　die Anordnung nach Fig. 1 für die Befüllung von Behältern mit anderem Durchmesser, ohne daß irgendeine Maßnahme an der Befüllungsvorrichtung erforderlich wäre; und

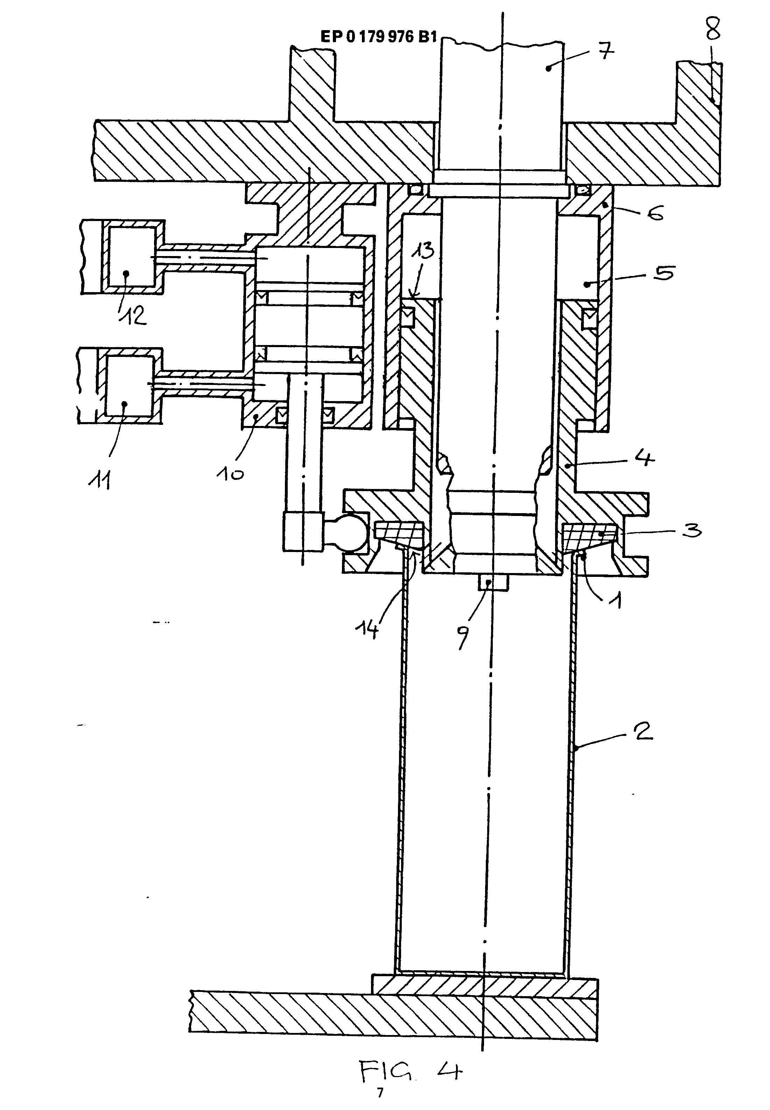

Fig. 4　die gleiche Anordnung wie in Fig. 1 zur Befüllung von Behältern mit anderer Höhe, wobei lediglich ein Eingriff bezüglich der vertikalen Lage des darüberliegenden Behälters erforderlich ist.

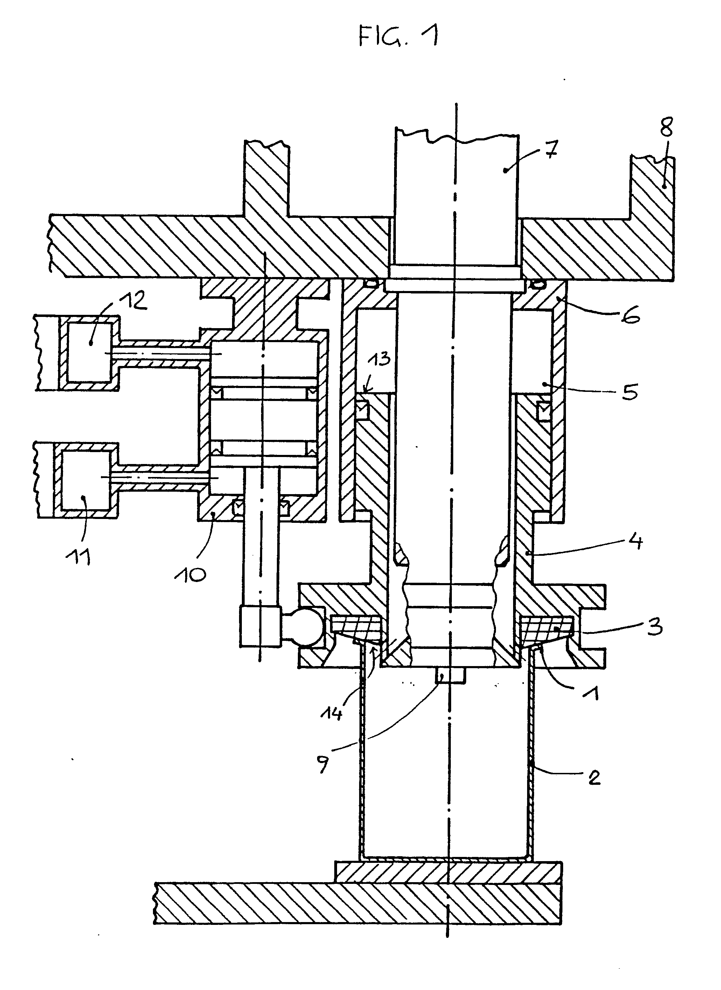

Aus Fig. 1 ist ersichtlich, daß vor dem Beginn des Füllvorganges ein Dichtring 3 auf den Rand 1 der Einfüllöffnung eines zylindrischen Behälters 2 aufgesetzt werden muß. Der Dichtring 3 ist in einen Schlitten 4 eingebaut der axial in einer zylindrischen Kammer 5 eines festen Teils 6 einer auf einen drehbaren Behälter 8 aufgesetzten Einfüllöffnung 7 beweglich ist. Mit dem Bezugszeichen 9 ist eine Kanüle bezeichnet, die ins Innere des Behälters 2 eindringt.

Vor dem Beginn des Füllvorganges muß der Dichtring mit einer ausreichenden Kraft gegen den Rand 1 gedrückt werden, um die Dichtigkeit zu gewährleisten, die unerläßlich ist, um die Einführung der Flüssigkeit durch die Kanüle 9 in den Behälter 2 zu beginnen; die Größe dieser Druckkraft muß jedoch gering sein, um den Rand 1 der Einfüllöffnung nicht zu beschädigen.

Zu Beginn wird diese geringe Kraft lediglich von einem Pneumatikglied 10 geliefert, das auf den Schlitten 4 einwirkt. Die einzufüllende Flüssigkeit wird in dieser Phase deshalb nicht wirksam, weil die Befüllung noch nicht begonnen hat. Auch wenn vor dem Füllvorgang abgesaugt wird, beispielsweise über die Kanüle 9, wird die erforderliche Mindestkraft für die Sicherung der Dichtigkeit lediglich von dem Pneumatikglied 10 erbracht. Die Druckverteilung im Inneren des Pneumatikgliedes 10 wird dadurch geregelt, daß die Betriebsdrücke der zugeordneten Druckluftbehälter 11 und 12 gesteuert werden. Die drehbaren Behälter sind im geschlossenen Kreis mit den rotierenden pneumatischen Pneumatikgliedern verbunden. Das Verhältnis zwischen den Drücken der Behälter 11 und 12 bestimmt den Wert und die Richtung der axial wirkenden Kraft auf den Rand 1. Der Wert des Verhältnisses der Drücke in den Druckluftbehältern ist auch während der kontinuierlichen Rotation mit hoher Geschwindigkeit regulierbar.

Anschließend wird die einzufüllende Flüssigkeit durch die Kanüle 9 in den Behälter 2 eingeführt und indem sie gleichzeitig in zweckmäßiger Weise in die Kammer 5 eindringt, wird auf den Schlitten 4 ein Axialschub ausgeübt, dessen Wert und Richtung von dem Verhältnis zwischen den aktiven Flächen von Schultern 13 und 14 abhängen. Die aktive Fläche der Schulter 13 ist konstant, während die aktive Fläche der Schulter 14 von dem Durchmesser des zu befüllenden Behälters abhängt; daraus folgt, daß der resultierende algebraische Wert des auf der einzufüllenden Flüssigkeit beruhenden Schubs durch die durch das Pneumatikglied gelieferte Hilfsschubkraft korrigiert werden muß. Um die Dichtschubkraft als Funktion des Durchmessers des zu füllenden Behälters und des Drucks der einzufüllenden Flüssigkeit zu optimieren, muß lediglich auf das Verhältnis der Drücke des Fluids, das in den jeweiligen rotierenden Druckluftbehältern enthalten ist, Einfluß genommen werden. Desgleichen muß auf das Verhältnis der Drücke in den Behältern 11 und 12 dann eingewirkt werden, wenn höhere oder dünnwandigere Behälter zu füllen sind, um deren Zusammenfallen während des Befüllens oder der eventuellen vorher erfolgenden Absaugung zu vermeiden. Die Regelung und die Steuerung des Druckverhältnisses in den Behältern 11 und 12 kann auch während der schnellen Rotation der kontinuierlichen Befüllungsvorrichtung mit größter Zuverlässigkeit und Unkompliziertheit erfolgen.

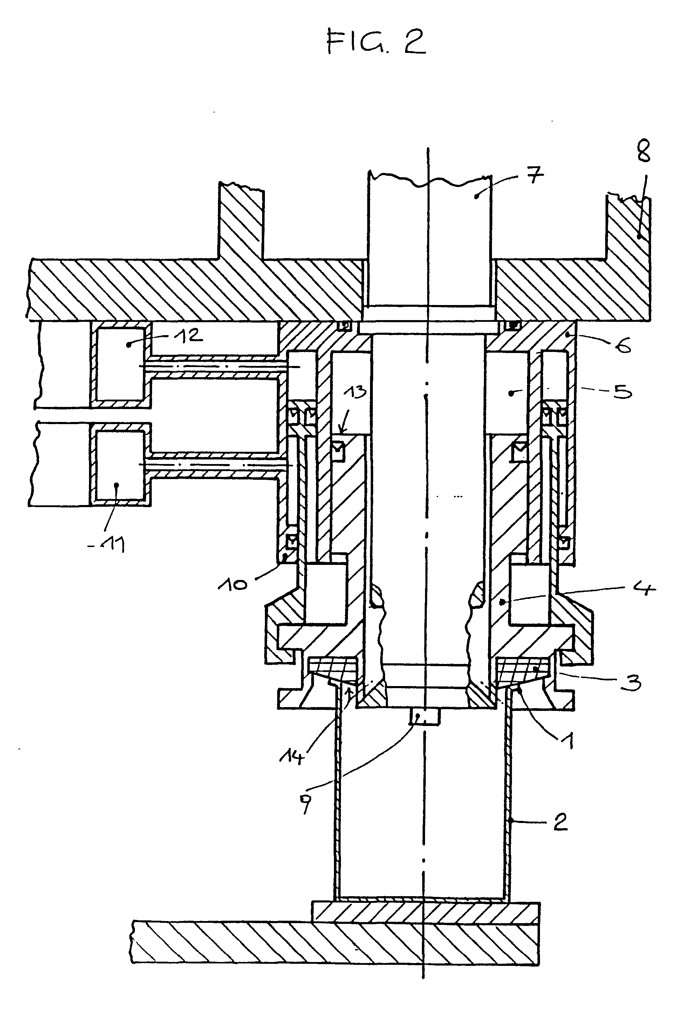

Bei der in Fig. 2 gezeigten Ausführungsform ist das Pneumatikglied 10 koaxial zu der Kammer 5 und dem Schlitten 4 angeordnet. Entsprechende Teile sind mit den gleichen Bezugszeichen wie in Fig. 1 versehen.

**Patentansprüche**

1. Kontinuierlich arbeitende Befüllungsvorrichtung mit einem rotierenden Tisch und mit einer Mehrzahl von Füllköpfen, die über Pneumatikglieder (10) mit dem Rand des zu füllenden Behälters dichtend in Anlage bringbar sind, dadurch gekennzeichnet, daß die Pneumatikglieder (10) mit Druckluftbehältern (11, 12) verbunden sind, deren Innendruckverhältnis gesteuert und vorherbestimmbar ist, indem nur auf ein einziges zentrales, dieses steuerndes Bauteil eingewirkt wird.

2. Befüllungsvorrichtung nach Anspruch 1, dadurch gekennzeichnet, daß sich der rotierende Tisch in fester Höhe befindet.

3. Befüllungsvorrichtung nach Anspruch 1 oder 2, dadurch gekennzeichnet, daß das Pneumatikglied (10) koaxial zu dem Befüllungskopf (4, 5, 6) angeordnet ist.

**Claims**

1. A continuously operating filling apparatus comprising a rotating table and a plurality of filling heads, which are adapted to be moved by pneumatic element, (10) into sealing engagement with the rim of the container that is to be filled, characterized in that the pneumatic elements (10) are connected to compressed air containers (11, 12), the internal pressure ratio of which is controlled and adapted to be predetermined by an action of a single central component for controlling that ratio.

2. A filling apparatus according to claim 1, characterized in that the rotating table is on a fixed level.

3. A filling apparatus according to claim 1 or 2, characterized in that the pneumatic element (10) is coaxial to the filling head (4, 5, 6).

**Revendications**

1. Dispositif de remplissage travaillant en continu comportant une table rotative et une pluralité de têtes de remplissage qui peuvent être amenées en contact étanche avec le bord du récipient à remplir au moyen d'éléments pneumatiques (10), caractérisé en ce que les éléments pneumatiques (10) sont raccordés à des récipients à air comprimé (11, 12) dont la pression intérieure est commandée et préprogrammable par l'action sur un seul composant central commandant la pression intérieure.

2. Dispositif de remplissage selon la revendication 1, caractérisé en ce que la table rotative est à une hauteur fixe.

3. Dispositif de remplissage selon les revendications 1 ou 2, caractérisé en ce que l'élément pneumatique (10) est disposé de manière coaxiale par rapport à la tête de remplissage (4, 5, 6).

FIG. 1

FIG. 2

FIG. 3

8

7

6

5

13

12

4

11

10

3

14

1

9

2

FIG. 4